# EUROPEAN PATENT APPLICATION

(11) **EP 4 588 491 A1**
(43) Date of publication of application: **23.07.2025**
(21) Application number: 23865550.0
(22) Date of filing: 13.09.2023
(51) Int. Cl.: A61K 51/04, A61K 51/10, A61P 35/00

(54) **PRODUCTION METHOD OF INTERMEDIATE FOR RADIOPHARMACEUTICAL COMPOSITION, AND PURIFICATION KIT FOR INTERMEDIATE FOR RADIOPHARMACEUTICAL COMPOSITION**

(30) Priority: 16.09.2022 JP 2022148406
(71) Applicant: National Institutes for Quantum Science and Technology, Chiba 263-8555 (JP)
(72) Inventor: YOSHII, Yukie, Chiba-shi, Chiba 263-8555 (JP); MATSUMOTO, Hiroki, Chiba-shi, Chiba 263-8555 (JP)
(74) Representative: Plasseraud IP
(86) International application number: PCT/JP2023/033315
(87) International publication number: WO 2024/058201

(57) **Abstract**

Production of a higher-purity intermediate of a radioactive antibody drug. The production method of the present invention includes: an adsorption step of adsorbing a composition containing the intermediate onto a stationary phase for hydrophobic chromatography; and a purification step of eluting the intermediate from the stationary phase. The intermediate is a complex of a 3,6,9,15-tetraazabicyclo[9.3.1]pentadeca-1(15),11,13-triene-3,6,9-triacetic acid (PCTA) and an anti-epidermal growth factor receptor antibody (anti-EGFR antibody).

## Description

### Technical Field

The present invention relates to a production method for an intermediate of a radiopharmaceutical composition, a radiopharmaceutical composition production method including the step of producing the intermediate by the production method, and a purification kit for an intermediate of a radiopharmaceutical composition.

### Background Art

Radioactive antibody drugs which are obtained by labeling with a radionuclide with use of a bifunctional chelator is under research and development. The bifunctional chelator is, for example, a 3,6,9,15-tetraazabicyclo[9.3.1]pentadeca-1(15),11,13-triene-3,6,9-triacetic acid (PCTA). For example, Non-patent Literatures 1 to 5 each disclose a method for radioimmunotherapy and diagnosis with use of a radioactive antibody drug.

### Citation List

### [Non-patent Literature]

[Non-patent Literature 1]
   Yoshii et al., Sci Rep 2020;10:4143
[Non-patent Literature 2]
   Matsumoto et al., Pharmaceutics. 2022;14:67.
[Non-patent Literature 3]
   Matsumoto et al., Pharmaceuticals. 2021;14:950.
[Non-patent Literature 4]
   Song IH et al., Oncotarget. 2017;8:92090-92105
[Non-patent Literature 5]
   Song IH et al., J Nucl Med. 2016;57: 1105-11

### Summary of Invention

### Technical Problem

A radioactive antibody drug is produced by coordinating a radioactive metal with an antibody-ligand complex obtained by combining an antibody and a bifunctional ligand. After being produced, an antibody and an antibody-ligand complex that serve as raw materials of an radioactive antibody drug undergo a purification step carried out by ultrafiltration, ion exchange chromatography, or the like, and then are used for production of the radioactive antibody drug.

Meanwhile, there is concern that such an antibody and such an antibody-ligand complex may reduce quality of a radioactive antibody drug due to a related substance generated during storage, and prevent practical use of the radioactive antibody drug. Examples of the related substance include a large-molecule related substance such as an aggregated dimer or multimer and a small-molecule related substance obtained by a breakage of an intramolecular bond of an antibody. However, a purification step carried out by conventional ultrafiltration, ion exchange chromatography, or the like makes it impossible to separate and purify (i) an antibody and an antibody-ligand complex, which are target substances, and (ii) such related substances. Thus, development of a technology for producing a higher-purity raw material of a radioactive antibody drug is desired.

In view of the above problems, an object of an aspect of the present invention is to provide a technology for producing a higher-purity intermediate of a radioactive antibody drug.

### Solution to Problem

As a result of making diligent studies, the inventors of the present invention have accomplished the present invention by finding the following: a high-purity intermediate of a radioactive antibody drug is obtained stably and at low cost by purifying an intermediate of a radioactive antibody drug by hydrophobic chromatography with use of a buffer containing a stabilizer as a mobile phase.

Specifically, in order to attain the object, a method for producing an intermediate of a radiopharmaceutical composition in accordance with an aspect of the present invention includes: an adsorption step of adsorbing a composition containing the intermediate onto a stationary phase for hydrophobic chromatography; and a purification step of eluting the intermediate from the stationary phase by passing an acetate buffer through the stationary phase, the acetate buffer serving as an eluent and containing at least one stabilizer selected from the group consisting of neutral amino acids and polysorbates, wherein the intermediate is a complex of a 3,6,9,15-tetraazabicyclo[9.3.1]pentadeca-1(15),11,13-triene-3,6,9-triacetic acid (PCTA) and an anti-epidermal growth factor receptor antibody (anti-EGFR antibody).

Further, a method for producing a radiopharmaceutical composition in accordance with an aspect of the present invention includes: the step of producing an intermediate by the method described above; and the step of labeling the intermediate with a radionuclide that coordinates with PCTA.

A kit for purification of an intermediate of a radiopharmaceutical composition in accordance with an aspect of the present invention includes: a stationary phase for hydrophobic chromatography; and an eluent containing at least one stabilizer selected from the group consisting of neutral amino acids and polysorbates, wherein the intermediate is a complex of a 3,6,9,15-tetraazabicyclo[9.3.1]pentadeca-1(15),11,13-triene-3,6,9-triacetic acid (PCTA) and an anti-epidermal growth factor receptor antibody (anti-EGFR antibody).

### Advantageous Effects of Invention

An aspect of the present invention makes it possible to produce a higher-purity intermediate of a radioactive antibody drug.

### Brief Description of Drawings

Fig. 1 is a diagram illustrating a result of analysis of PCTA-cetuximab by size exclusion chromatography before separation and purification of the PCTA-cetuximab by hydrophobic chromatography, in accordance with Examples of the present invention.
Fig. 2 is a diagram illustrating a result of size exclusion chromatography analysis of a fraction 2, which was collected by hydrophobic chromatography from PCTA-cetuximab, in accordance with Examples of the present invention.
Fig. 3 is a diagram illustrating a result of size exclusion chromatography analysis of a fraction 9, which was collected by hydrophobic chromatography from PCTA-cetuximab, in accordance with Examples of the present invention.
Fig. 4 is a diagram illustrating a result of size exclusion chromatography analysis of a fraction 16, which was collected by hydrophobic chromatography from PCTA-cetuximab, in accordance with Examples of the present invention.
Fig. 5 is a diagram illustrating a result of analysis of PCTA-cetuximab by size exclusion chromatography carried out at the start of cold storage of the PCTA-cetuximab, the PCTA-cetuximab having been separated and purified by hydrophobic chromatography, in accordance with Examples of the present invention.
Fig. 6 is a diagram illustrating a result of analysis of PCTA-cetuximab by size exclusion chromatography carried out after 1 month of cold storage of the PCTA-cetuximab, the PCTA-cetuximab having been separated and purified by hydrophobic chromatography, in accordance with Examples of the present invention.
Fig. 7 is a diagram illustrating a result of analysis of PCTA-cetuximab by size exclusion chromatography carried out after 3 months of cold storage of the PCTA-cetuximab, the PCTA-cetuximab having been separated and purified by hydrophobic chromatography, in accordance with Examples of the present invention.
Fig. 8 is a diagram illustrating a result of analysis of PCTA-cetuximab by size exclusion chromatography carried out after 6 months of cold storage of the PCTA-cetuximab, the PCTA-cetuximab having been separated and purified by hydrophobic chromatography, in accordance with Examples of the present invention.

### Description of Embodiments

[Method for producing intermediate of radiopharmaceutical composition]

In a method for producing an intermediate of a radiopharmaceutical composition in accordance with an aspect of the present invention (hereinafter sometimes abbreviated as "a method for producing an intermediate in accordance with the present embodiment"), a related substance of an intermediate of the radiopharmaceutical composition is separated and removed by an adsorption step and a purification step, which will be described later, so that a high-purity intermediate can be obtained.

### (Radiopharmaceutical composition and intermediate thereof)

In the present specification, the radiopharmaceutical composition refers to a complex of a 3,6,9,15-tetraazabicyclo[9.3.1]pentadeca-1(15),11,13-triene-3,6,9-triacetic acid (PCTA) and an anti-epidermal growth factor receptor antibody (anti-EGFR antibody). The complex is labeled with a radionuclide. The radiopharmaceutical composition can be used in diagnosis or treatment of a disease. Further, in the present specification, the intermediate of the radiopharmaceutical composition refers to a pharmaceutical composition prior to labeling with a radionuclide (that is, a complex of PCTA and an anti-EGFR antibody, which is not labeled with the radionuclide). The radionuclide will be discussed later.

### (PCTA)

The PCTA is a bifunctional chelator. Into the PCTA, an isocyanate group or an N-hydroxysuccinimidyl group is introduced. Then, on a matter thus obtained, a side-chain amino group or an N-terminal amino group of a lysine residue of an antibody is caused to act, so that a chelating site is introduced into the antibody. Thereafter, at the chelating site, complex forming of the radionuclide is carried out, so that an antibody labeled with the radionuclide can be obtained.

### (Anti-EGFR antibody)

Examples of the anti-EGFR antibody include an antibody that specifically binds to cancer. Specific examples of the anti-EGFR antibody include cetuximab, panitumumab, and necitumumab. The anti-EGFR antibody may be a mouse antibody, a chimeric antibody, a humanized antibody, or a human antibody. Further, the anti-EGFR antibody may be a monoclonal antibody or a polyclonal antibody. The anti-EGFR antibody used can be a commercially available anti-EGFR antibody or an anti-EGFR antibody synthesized by a well-known method.

The intermediate used may be an intermediate synthesized by a well-known method. For example, as described in Examples, the intermediate can be obtained by: mixing a solution containing the PCTA and a solution containing the anti-EGFR antibody together; and incubating a resultant mixture at 1°C to 60°C for 1 hour to 24 hours.

Examples of the related substance of the intermediate of the radiopharmaceutical composition include a large-molecule related substance (such as a dimer of the intermediate or a multimer of the intermediate), and a small-molecule related substance obtained by a breakage of an intramolecular bond of an antibody in the intermediate.

### (Adsorption step)

In the adsorption step, a composition containing the intermediate is adsorbed onto a stationary phase for hydrophobic chromatography. Examples of the composition containing the intermediate include a composition containing the intermediate immediately after synthesis and a composition containing the intermediate stored for a certain period of time after synthesis. The composition containing the intermediate contains not only the intermediate but also, for example, a related substance of the intermediate.

The stationary phase for hydrophobic chromatography can be, for example, a hydrophilic polymer substrate to which a cyclic or linear lipophilic functional group is bound. Examples of the hydrophilic polymer substrate include hydrophilic polymer substrates used in well-known hydrophobic chromatography (e.g., polysaccharides, synthetic resins such as a vinyl polymer, silica, etc.). The hydrophilic polymer substrate may be porous or non-porous. Further, the shape of the hydrophilic polymer substrate is not particularly limited, and may be, for example, spherical. The lipophilic functional group may be directly bound to a hydrophilic polymer or indirectly bound to the hydrophilic polymer via a linker.

The cyclic lipophilic functional group may have not less than 6 carbon atoms or may have not more than 20 carbon atoms. Further, the cyclic lipophilic functional group may be an aryl group, and is preferably an aryl group having 6 to 20 carbon atoms because such an aryl group makes it possible to obtain a higher-purity intermediate. Examples of the aryl group having 6 to 20 carbon atoms include a phenyl group and a naphthyl group. Examples of the hydrophilic polymer substrate to which an aryl group having 6 to 20 carbon atoms is bound include TOYOPEARL (registered trademark) Phenyl, HiPrep (registered trademark) Phenyl FF, POROS (registered trademark) Benzyl, and POROS (registered trademark) Benzyl Ultra.

The linear lipophilic functional group may have not less than 1 carbon atom or may have not more than 10 carbon atoms. Further, the linear lipophilic functional group may be an alkyl group. Examples of the linear lipophilic functional group having 1 to 10 carbon atoms include a methyl group, an ethyl group, a propyl group, an isopropyl group, a butyl group, a hexyl group, and an octyl group. Examples of the hydrophilic polymer substrate to which a linear alkyl group having 1 to 10 carbon atoms is bound include TOYOPEARL (registered trademark) Butyl, TOYOPEARL (registered trademark) Hexyl, HiPrep (registered trademark) Butyl FF, and HiPrep (registered trademark).

The composition containing the intermediate may be adsorbed onto the stationary phase by, for example, passing a solution containing the composition through a column filled with a stationary phase, and bringing the composition into contact with the stationary phase.

### (Purification step)

In the purification step, the intermediate is eluted from the stationary phase by passing, through the stationary phase on which the composition has been adsorbed, a buffer serving as an eluent (mobile phase).

The buffer used as the eluent in the purification step is an acetate buffer. The acetate buffer preferably has a pH of 5 to 8.

The eluent contains at least one stabilizer selected from the group consisting of neutral amino acids and polysorbates.

The eluent preferably contains a neutral amino acid as the stabilizer in terms of, for example, having radical-scavenging activity. Among neutral amino acids, a neutral amino acid having high solubility is more preferable in terms of, for example, increasing stability of the antibody eluted in the eluent. Examples of the neutral amino acid having high solubility include asparagine, glutamine, serine, threonine, cysteine, alanine and glycine. One type of the above neutral amino acids may be contained in the eluent, or two or more types of the above neutral amino acids may be contained in the eluent.

The concentration of the neutral amino acid contained in the eluent is preferably not less than 1 µM, more preferably not less than 10 pM, and even more preferably not less than 50 pM, in terms of, for example, increasing the stability of the antibody eluted in the eluent. Further, the concentration of the neutral amino acid contained in the eluent is preferably not more than 260 mM, more preferably not more than 200 mM, and even more preferably not more than 150 mM.

The eluent preferably contains polysorbate because, for example, containing polysorbate allows the eluent to have radical-scavenging activity and to stably elute the intermediate into the eluent by suppressing antibody (protein) aggregation. Examples of the polysorbate include polysorbate 20, polysorbate 40, polysorbate 60, polysorbate 65, and polysorbate 80. One type of the above polysorbates may be contained in the eluent, or two or more types of the above polysorbates may be contained in the eluent.

The concentration of the polysorbate contained in the eluent is preferably not less than 1 µM, more preferably not less than 10 µM, and even more preferably not less than 50 pM, because at such a concentration, protein aggregation is suppressed and the intermediate can be stably eluted in the eluent. Further, the concentration of the polysorbate contained in the eluent is preferably not more than 400 mM, more preferably not more than 200 mM, and even more preferably not more than 100 mM.

The eluent preferably contains at least one type of neutral amino acid and at least one type of polysorbate. In a case where the eluent, which is an acetate buffer, contains a neutral amino acid and a polysorbate, a synergistic effect of the acetate buffer, the neutral amino acid and the polysorbate can be exerted. Then, protein aggregation is further suppressed. As a result, the intermediate can be stably eluted in the eluent. This further provides the effect of suppressing radioactive degradation of the radiopharmaceutical composition prepared with use of the intermediate eluted in the eluent.

The method for producing an intermediate in accordance with the present embodiment makes it possible to remove a related substance from an intermediate by simple isocratic elution without a change in composition of an eluent.

The eluent containing salt may be further passed through the stationary phase, on which the composition has been adsorbed, after the eluent is passed through the stationary phase. Further passing the eluent containing salt through the stationary phase makes it possible to collect a component (a component other than the intermediate) that is adsorbed on the stationary phase. Examples of the salt include sodium chloride. The eluent containing salt may be passed at a constant concentration, or a mixed solution of the eluent and the eluent containing salt may be passed while a mixing ratio is changed.

### (Purity measurement step)

The method for producing an intermediate in accordance with the present embodiment may further include, after the purification step, a purity measurement step of measuring the purity of the intermediate. A purity measurement method may be a well-known method and can be, for example, a method carried out by size exclusion chromatography, or the like.

### (Storage step)

The method for producing an intermediate in accordance with the present embodiment may further include, after the purification step, a storage step of storing the eluent containing the intermediate for not less than 24 hours. Using the eluent as a stock solution makes it possible to suppress generation of a related substance of the intermediate during storage. The intermediate may be stored for a storage period of not less than 48 hours, not less than 1 week, or not less than 1 month. Further, the intermediate may be stored for a storage period of not more than 3 years, not more than 2 years, and not more than 12 months.

In the storage step, the storage temperature of the intermediate is preferably not less than 1°C, and more preferably not less than 3°C, in terms of, for example, stability of the intermediate. Further, the storage temperature of the intermediate is preferably not more than 8°C, and more preferably not more than 5°C.

The method for producing an intermediate in accordance with the present embodiment may include another step(s). Examples of such a step include the step of adjusting the concentration of the intermediate with use of, for example, an ultrafiltration membrane.

The acetate buffer used in the eluent can suppress protein aggregation and store the intermediate for a desired period of time. Further, the stabilizer contained in the eluent improves stability of the intermediate stored in a liquid. Thus, the eluent containing the intermediate obtained after the purification step can store the intermediate without carrying out solvent replacement.

In the prior art, an intermediate is purified by a purification method such as ultrafiltration or ion exchange chromatography. However, such a method makes it impossible to separate a target intermediate and a related substance (such as a dimer of the intermediate), and has a major problem in terms of, for example, a risk of failing to meet quality standards for radioactive antibody pharmaceutical products in practical applications. In contrast, the method for producing an intermediate in accordance with the present embodiment makes it possible to efficiently remove, by a single step (purification step), a related substance such as an aggregate or a fragment. Thus, using the method for producing an intermediate in accordance with the present embodiment makes it possible to reduce a risk of an intermediate, which is a target compound, failing to meet a quality standard, and to use a stored product for a long term by separating and purifying the stored product. Further, solvent replacement after purification is unnecessary. This makes it possible to achieve stable and low-cost production of a radioactive antibody drug. In addition, a method for storing an intermediate in accordance with the present embodiment can conform to the pharmaceutical good manufacturing practice (GMP).

### [Purification kit for intermediate of radiopharmaceutical composition]

An aspect of the present invention also encompasses a purification kit for purifying an intermediate in accordance with the present embodiment. The purification kit includes: a stationary phase for hydrophobic chromatography; and an eluent containing at least one stabilizer selected from the group consisting of neutral amino acids and polysorbates.

The purification kit may include, in addition to the stationary phase and the eluent, other materials such as a salt such as sodium chloride and a column to be filled with the stationary phase. The stationary phase may be filled in the column in advance. Further, the eluent may be two types of eluents: an eluent containing at least one stabilizer selected from the group consisting of neutral amino acids and polysorbates; and the eluent containing a salt such as sodium chloride.

Further, the purification kit may include an instruction manual describing, for example, a procedure for purifying the intermediate. The instruction manual may be written or printed on paper or another medium, or may be attached to a magnetic tape or an electronic medium readable by, for example, a computer, such as a disk or a CD-ROM. The purification kit may include, instead of the instruction manual, a voice file or a video file explaining, for example, the procedure for purifying the intermediate. The voice file or the video file may be attached to a magnetic tape or an electronic medium readable by, for example, a computer, such as a disk or a CD-ROM, or may be stored in, for example, an accessible cloud server in the form of being referred to via an internet line.

### [Method for producing radiopharmaceutical composition]

A method for producing a radiopharmaceutical composition in accordance with an aspect of the present invention includes a labeling step of labeling, with a radionuclide that coordinates with PCTA, the intermediate produced by the method for producing an intermediate.

### (Labeling step)

The radionuclide used in the labeling step is a radionuclide that coordinates with PCTA. Examples of the radionuclide include ⁶⁴Cu and ²²⁵Ac.

The intermediate can be labeled with the radionuclide by a well-known method. For example, as shown in Examples, the stock solution containing the intermediate is mixed with a solution containing the radionuclide. Further, a resultant mixture is incubated at 1°C to 60°C for 5 minutes to 12 hours. This makes it possible to label the intermediate with the radionuclide. The solution containing the radionuclide is preferably an acetate buffer, and more preferably an acetate buffer having the same pH as the stock solution.

The preparation method in accordance with the present embodiment for the radiopharmaceutical composition may include another step in addition to the labeling step. Examples of such a step include the step of measuring radiochemical purity of the radiopharmaceutical composition.

Aspects of the present invention can also be expressed as follows:
A method for producing an intermediate of a radiopharmaceutical composition in accordance with an aspect of the present invention includes: an adsorption step of adsorbing a composition containing the intermediate onto a stationary phase for hydrophobic chromatography; and a purification step of eluting the intermediate from the stationary phase by passing an acetate buffer through the stationary phase, the acetate buffer serving as an eluent and containing at least one stabilizer selected from the group consisting of neutral amino acids and polysorbates, wherein the intermediate is a complex of a 3,6,9,15-tetraazabicyclo[9.3.1]pentadeca-1(15),11,13-triene-3,6,9-triacetic acid (PCTA) and an anti-epidermal growth factor receptor antibody (anti-EGFR antibody).

In the method for producing an intermediate in accordance with an aspect of the present invention, the stabilizer may be at least one type of neutral amino acid and at least one type of polysorbate.

In the method for producing an intermediate in accordance with an aspect of the present invention, the stationary phase may be a hydrophilic polymer substrate to which a cyclic or linear lipophilic functional group is bound.

In the method for producing an intermediate in accordance with an aspect of the present invention, after the eluent is passed through the stationary phase, the eluent containing sodium chloride may be further passed through the stationary phase.

In the method for producing an intermediate in accordance with an aspect of the present invention, the anti-EGFR antibody may be cetuximab.

The method for producing an intermediate in accordance with an aspect of the present invention may further include, after the purification step, a purity measurement step of measuring the purity of the intermediate using size exclusion chromatography.

The method for producing an intermediate in accordance with an aspect of the present invention may further include, after the purification step, a storage step of storing the eluent containing the intermediate for not less than 24 hours.

A method for producing a radiopharmaceutical composition in accordance with an aspect of the present invention includes: the step of producing an intermediate by the method for producing an intermediate; and the step of labeling the intermediate with a radionuclide that coordinates with PCTA.

A kit for purification of an intermediate of a radiopharmaceutical composition in accordance with an aspect of the present invention includes: a stationary phase for hydrophobic chromatography; and an eluent containing at least one stabilizer selected from the group consisting of neutral amino acids and polysorbates, wherein the intermediate is a complex of a 3,6,9,15-tetraazabicyclo[9.3.1]pentadeca-1(15),11,13-triene-3,6,9-triacetic acid (PCTA) and an anti-epidermal growth factor receptor antibody (anti-EGFR antibody).

The following description will more specifically discuss an embodiment of the present invention with reference to Examples. The present invention is, of course, not limited to the Examples below and particulars can have various aspects. Further, the present invention is not limited to the above-described embodiments, but can be variously altered by a person skilled in the art within the scope of the claims. An embodiment derived from a proper combination of technical means disclosed in respective different embodiments is also encompassed in the technical scope of the present invention. Moreover, all the literatures described herein are hereby incorporated by reference.

### Examples

In the following Examples, "%" represents "% by mass".

### [Material and method]

### [Synthesis method for PCTA-cetuximab]

Cetuximab, which is an EGFR antibody, was obtained by a well-known method. Specifically, a DNA strand obtained with use of the gene sequence of cetuximab and encoding cetuximab was transfected into Chinese hamster ovary (CHO) cells. Cetuximab thus synthesized was purified.

According to the description in Nucl Med Biol 43 (11): 685-691, ⁶⁴Cu was prepared with use of a cyclotron and purified. In order to form a coordination with ⁶⁴Cu and form a bond with cetuximab, 3,6,9,15-Tetraazabicyclo[9.3.1]pentadeca-1(15),11,13-triene-4-S-(4-isothiocyanatobenzyl)-3,6,9-triacetic acid (p-SCN-Bn-PCTA, Macrocyclics, Plano, TX, USA) was used. Sterilized water used was water of a pharmaceutical grade, while the other chemicals used were chemicals of a chemical grade.

Formation of a complex of cetuximab and PCTA was carried out by methods which were described in the following literatures and to which several changes were made: Cancer Sci 103 (3): 600-605; PLoS One 8 (4): e61230; and PLoS One 10 (4): e0123761. Briefly, a borate buffer (0.05 M, pH 8.5) solution (2 mg/mL) of cetuximab was prepared by replacing the buffer with use of a Vivaspin ultrafiltration device (Sartorius, Aubagne, France). The borate buffer of cetuximab was mixed with a dimethyl sulfoxide solution (0.42 mg/mL) of p-SCN-Bn-PCTA at a ratio of 10:1 (cetuximab : p-SCN-Bn-PCTA, v/v). Then, a reaction was carried out at 37°C for 24 hours, so that PCTA-cetuximab was prepared. The PCTA-cetuximab thus prepared was subjected to solution replacement to a concentration of 2 mg/mL using an ultrafiltration centrifugal concentrator tube with use of 0.1 M acetate buffer (pH 6.0) containing 100 mM glycine and 76.3 µM polysorbate 80.

### [Analysis method for chemical purity of PCTA-cetuximab]

Chemical purity of the PCTA-cetuximab prepared above was analyzed with use of size exclusion chromatography (SEC-HPLC). A column used was TSKgel G3000 SWXL (300×7.8 mm, 5 µm, manufactured by Tosoh Corporation), and a guard column used was Security Guard Cartridges GFC-3000 (manufactured by Phenomex). A mobile phase used was a potassium phosphate buffer containing 250 mM KCl. Analysis was carried out at a flow rate of 0.5 mL/min in the mobile phase, and a detection wavelength was set at 280 nm.

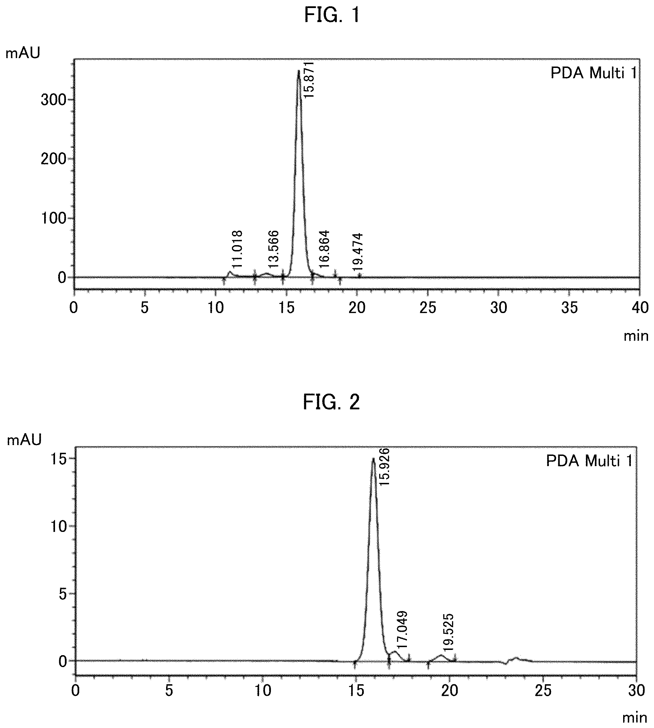

Fig. 1 shows typical results. In the prepared PCTA-cetuximab, a target compound (PCTA-cetuximab) was detected at a retention time of 15.871 minutes. In contrast, related substances with larger molecular sizes than the target compound were detected at 11.018 minutes and 13.566 minutes, and related substances with smaller molecular sizes than the target compound were detected at 16.864 minutes and 19.474 minutes.

### (Example 1) Method for removing related substance from PCTA-cetuximab with use of hydrophobic chromatography

The prepared PCTA-cetuximab was separated and purified by hydrophobic chromatography. A column used was TOYOPEARL (registered trademark) Phenyl FT-750F (1.0 cm×6.0 cm, bed: 4.7 mL, manufactured by Tosoh Corporation). A mobile phase A used was a 0.1 M acetate buffer (pH 6.0) containing 100 mM glycine and 76.3 µM polysorbate 80 as stabilizers. A mobile phase B used was obtained by adding 1 M sodium chloride to the mobile phase A. Into the column, 5 mL of a PCTA-cetuximab solution (2 mg/mL) was injected. Next, 10 column volume of the mobile phase A was flowed, and then a ratio of the mobile phase B was increased over 8 column volumes to 100%. A flow rate was 2.5 mL/min (linear flow rate: 191 cm/h), a detection wavelength was 280 nm, and an amount of fraction fractionated was 5 mL.

For each fraction collected, chemical purity of the PCTA-cetuximab contained in the fraction was analyzed by size exclusion chromatography. Analysis was carried out under a condition similar to that described in [Analysis method for chemical purity of PCTA-cetuximab] above.

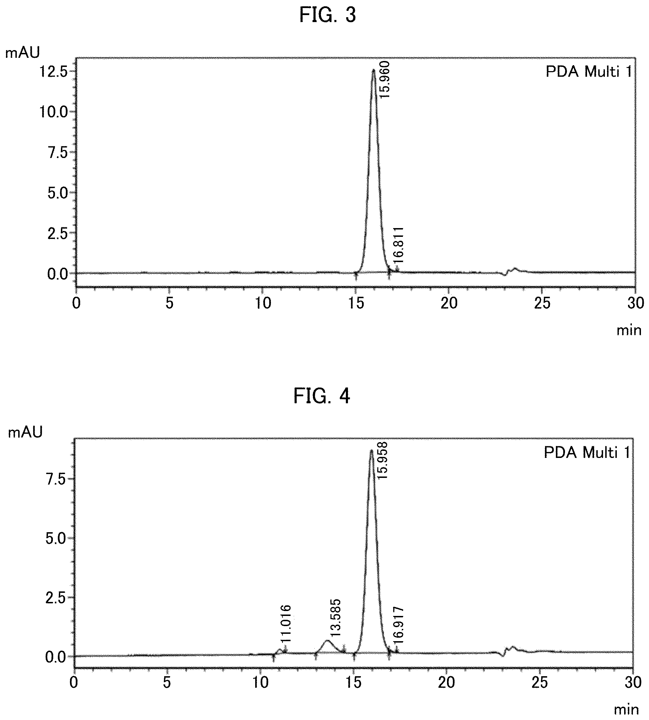

Figs. 2 to 4 show typical results. Fig. 2 is a diagram illustrating a result of size exclusion chromatography analysis of a fraction 2, which was collected by hydrophobic chromatography from the prepared PCTA-cetuximab. Fig. 3 is a diagram illustrating a result of size exclusion chromatography analysis of a fraction 9, which was collected by hydrophobic chromatography from the prepared PCTA-cetuximab. Fig. 4 is a diagram illustrating a result of size exclusion chromatography analysis of a fraction 16, which was collected by hydrophobic chromatography from the prepared PCTA-cetuximab. In fractions 1 to 3, a target compound and related substances with smaller molecular sizes than the target compound were detected. In fractions 4 to 9, only a target compound containing almost no related substance was detected. In a fraction 10 and subsequent fractions, a target compound and related substances with larger molecular sizes than the target compound were detected. Thus, it has been determined that collecting the fractions 4 to 9 makes it possible to separate and purify the PCTA-cetuximab which contains almost no related substance.

### (Comparative Examples 1 to 4)

The prepared PCTA-cetuximab was separated and purified by hydrophobic chromatography. In each of Comparative Examples, a column used was TOYOPEARL (registered trademark) Phenyl FT-750F (1.0 cm×6.0 cm, bed: 4.7 mL, manufactured by Tosoh Corporation), and a mobile phase used was a combination of four types shown in Table 1. In each of Comparative Examples, the following mode (flow-through mode) was set: the mobile phase B and the mobile phase A were flowed into the column in this order, a sample was introduced into a resin equilibrated at a high salt concentration to collect a target compound in flow-through, and an aggregate was eluted by lowering the salt concentration. As a result, in all Comparative Examples 1 to 4, the target compound and the aggregate were eluted in the flow-through without being separated. A comparison between results of Examples and Comparative Example 3 has suggested that presence or absence of polysorbate is involved in retention of the aggregate by the column for a certain period of time.

**[Table 1]**

| Table 1: Condition of column chromatography separation and purification of PCTA-cetuximab | | | |
|---|---|---|---|
| (Flow-through mode, Comparative Examples 1 to 4) | | | |
| Comparative Example | Mobile phase A | Mobile phase B | Injected sample |
| 1 | 50 mM phosphate buffer pH 6.8 | Mobile phase A + 150 mM sodium sulfate | Addition of 300 mM aqueous sodium sulfate solution to PCTA-cetuximab so as to achieve final concentration of 150 mM |
| 2 | 50 mM acetate buffer pH 6.0 | Mobile phase A + 50 mM glycine | Dilution of PCTA-cetuximab solution 2 times with purified water |
| 3 | 100 mM acetate buffer pH 6.0 | Mobile phase A + 100 mM glycine | |
| 4 | 300 mM acetate buffer pH 6.0 | 50 mM acetate buffer pH 6.0 | Desalination through 50 kDa membrane |

### (Comparative Examples 5 to 7)

PCTA-cetuximab was separated and purified by anion exchange chromatography. A column used was HiTrap Q FF (1.6×2.5 cm, bed: 5 mL, manufactured by Cytiva), and a mobile phase used was a combination of two types shown in Table 2. In each of Comparative Examples, the following mode (desorption and adsorption mode) was set: the mobile phase A and the mobile phase B were flowed into the column in this order, a sample was introduced into a resin equilibrated at a low salt concentration to collect a target compound in flow-through, and an aggregate was eluted by raising the salt concentration. As a result, in Comparative Examples 5 and 6, the target compound and the aggregate were eluted in the flow-through and were not able to be separated. In Comparative Example 7, with a gradient fraction of the salt concentration, the target compound and the aggregate were eluted without being separated.

**[Table 2]**

| Table 2: Condition of column chromatography separation and purification of PCTA-cetuximab | | | |
|---|---|---|---|
| (Desorption and adsorption mode, Examples 5 to 7) | | | |
| Comparative Example | Mobile phase A | Mobile phase B | Injected sample |
| 5 | 0.1 M acetate buffer containing 100 mM glycine and 76.3 µM polysorbate 80 pH 6.0 | Mobile phase A + 1 M sodium chloride | PCTA-cetuximab solution |
| 6 | 50 mM Tris/HCl pH 8.0 | Mobile phase A + 1 M sodium chloride | PCTA-cetuximab solution |
| | | | Dilution 2 times with mobile phase A |
| 7 | 50 mM Tris/HCl pH 8.0 | Mobile phase A + 1 M sodium chloride | PCTA-cetuximab solution |
| | | | Dilution 3 times with mobile phase A |

### [Stability of purified PCTA-cetuximab]

Stability was confirmed for the PCTA-cetuximab that had been separated and purified by the method described in Example 1. The PCTA-cetuximab was subjected to cold storage at a storage temperature of 5°C±3°C. Then, at the start of storage, after 1 month of storage, after 3 months of storage, and after 6 months of storage, chemical purity of the PCTA-cetuximab was analyzed by size exclusion chromatography to evaluate stability of the PCTA-cetuximab. Analysis was carried out under a condition similar to that described in [Analysis method for chemical purity of PCTA-cetuximab] above.

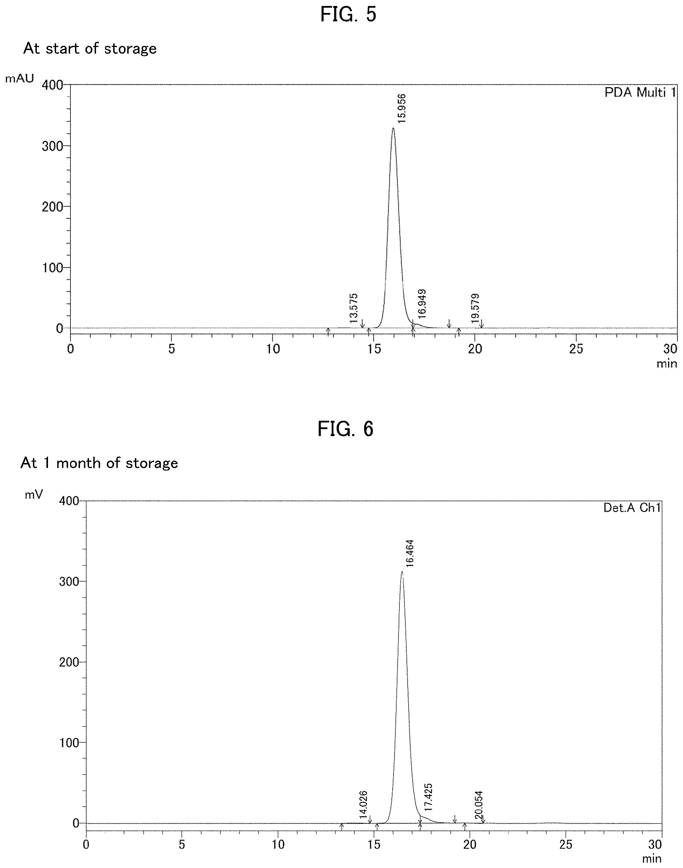

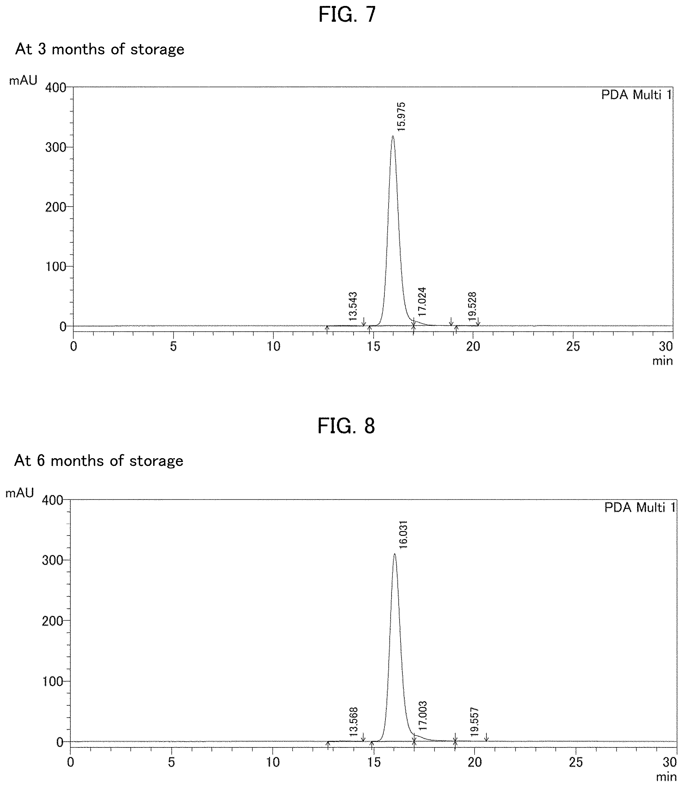

Figs. 5 to 8 show results. It has been confirmed that, although a related substance with a larger molecular size than a peak indicating the PCTA-cetuximab tended to slightly increase over time, chemical purity of the PCTA-cetuximab was maintained at not less than 96% even after 6 months from the start of storage. Thus, it has been concluded that the PCTA-cetuximab separated and purified by the method which is described in Example 1 and in which the hydrophobic chromatography was used was stable for 6 months in cold storage and that such a separation and purification method was practically useful.

### Industrial Applicability

The present invention can be used for stable and low-cost production of an intermediate of a radiopharmaceutical composition.

## Claims

1. A method for producing an intermediate of a radiopharmaceutical composition, comprising:
an adsorption step of adsorbing a composition containing the intermediate onto a stationary phase for hydrophobic chromatography; and
a purification step of eluting the intermediate from the stationary phase by passing an acetate buffer through the stationary phase, the acetate buffer serving as an eluent and containing at least one stabilizer selected from the group consisting of neutral amino acids and polysorbates,
wherein the intermediate is a complex of a 3,6,9,15-tetraazabicyclo[9.3.1]pentadeca-1(15),11,13-triene-3,6,9-triacetic acid (PCTA) and an anti-epidermal growth factor receptor antibody (anti-EGFR antibody).

2. The method according to claim 1, wherein the stabilizer is at least one type of neutral amino acid and at least one type of polysorbate.

3. The method according to claim 1, wherein the stationary phase is a hydrophilic polymer substrate to which a cyclic or linear lipophilic functional group is bound.

4. The method according to claim 3, wherein the cyclic lipophilic functional group is a functional group having 6 to 20 carbon atoms.

5. The method according to claim 3, wherein the linear lipophilic functional group is a functional group having 1 to 10 carbon atoms.

6. The method according to claim 1, wherein, after the eluent is passed through the stationary phase, the eluent containing sodium chloride is further passed through the stationary phase.

7. The method according to claim 1, wherein the anti-EGFR antibody is cetuximab.

8. The method according to claim 1, further comprising, after the purification step, a purity measurement step of measuring the purity of the intermediate using size exclusion chromatography.

9. The method according to claim 2, further comprising, after the purification step, a storage step of storing the eluent containing the intermediate for not less than 24 hours.

10. A method for producing a radiopharmaceutical composition, comprising:
the step of producing an intermediate by the method according to any one of claims 1 to 9; and
the step of labeling the intermediate with a radionuclide that coordinates with PCTA.

11. A kit for purification of an intermediate of a radiopharmaceutical composition, comprising:
a stationary phase for hydrophobic chromatography; and
an eluent containing at least one stabilizer selected from the group consisting of neutral amino acids and polysorbates,
wherein the intermediate is a complex of a 3,6,9,15-tetraazabicyclo[9.3.1]pentadeca-1(15),11,13-triene-3,6,9-triacetic acid (PCTA) and an anti-epidermal growth factor receptor antibody (anti-EGFR antibody).
